# FASCICULE DE BREVET EUROPEEN

(11) **EP 1 865 863 B1**
(45) Date de publication et mention de la délivrance du brevet: **29.02.2012**
(21) Numéro de dépôt: 06726310.3
(22) Date de dépôt: 06.04.2006
(51) Int. Cl.: A61B 17/70

(54) **IMPLANT INTERVERTEBRAL POUR L'ARTICULATION LOMBO-SACREE.**
INTERVERTEBRALES IMPLANTAT FÜR DAS LUMBROSAKRALGELENK
INTERVERTEBRAL IMPLANT FOR LUMBROSACRAL JOINT

(30) Priorité: 07.04.2005 FR 0503462
(43) Date de publication de la demande: 19.12.2007
(73) Titulaire: ZIMMER SPINE, 33000 Bordeaux (FR)
(72) Inventeur: PASQUET, Denis, 33360 Quinsac (FR)
(74) Mandataire: Intès, Didier Gérard André
(86) Numéro de dépôt international: PCT/FR2006/050304
(87) Numéro de publication internationale: WO 2006/106268

(56) Documents cités:
- EP-A- 1 138 268
- WO-A-2005/020860
- WO-A-2006/110578
- FR-A- 2 714 591
- FR-A- 2 799 948

## Description

La présente invention a pour objet un Implant intervertébral pour l'articulation lombo-sacrée.

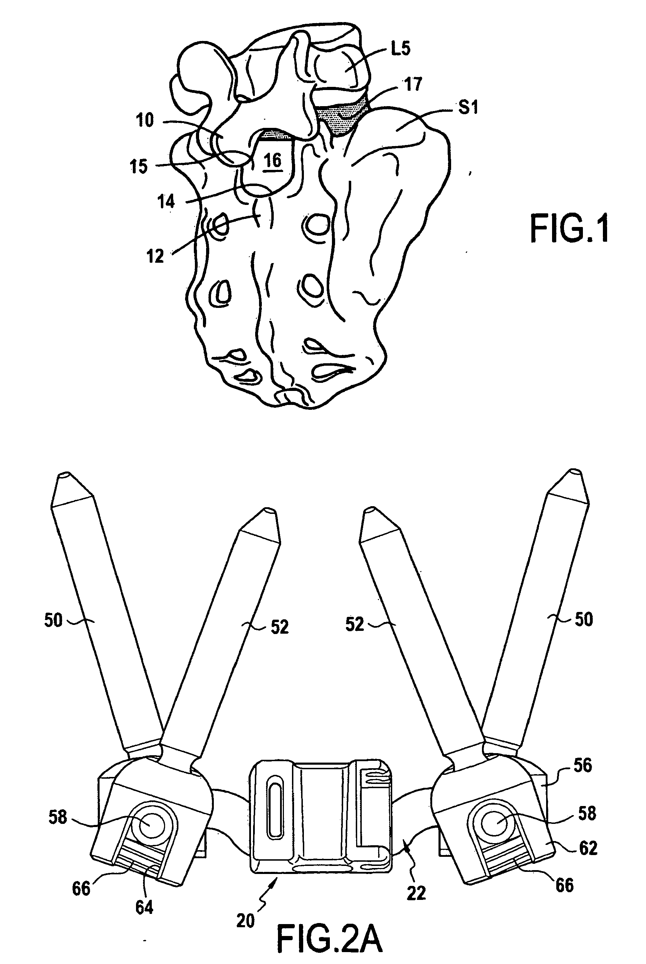

Dans l'anatomie du rachis, le sacrum, situé en dessous des vertèbres lombaires, est constitué par cinq vertèbres qui, au fil de l'évolution humaine, se sont soudées entre elles. La vertèbre supérieure du sacrum, notée S1, est articulée à la cinquième vertèbre lombaire, notée L5. Cette articulation constitue l'articulation lombo-sacrée, ou articulation L5-S1, représentée figure 1 annexée.

Les vertèbres lombaires présentent une saillie médiane et postérieure : l'apophyse épineuse, dénommée ci-après épineuse 10. Les vertèbres sacrées, elles, ont perdu leurs épineuses au fil de l'évolution, et ne conservent à la place qu'une petite bosse résiduelle 12.

Chez l'homme, certaines douleurs dorsales peuvent être dues aux contraintes, liées au déplacement relatif de deux vertèbres entre elles, qui s'exercent sur le disque intervertébral situé entre ces vertèbres.

On connaît déjà de nombreux implants intervertébraux qui visent à limiter le déplacement de deux vertèbres entre elles de manière à soulager le disque intervertébral, et notamment celui décrit dans le document FR 2 775 183. Cet implant est une cale présentant sur ces faces supérieure et inférieure deux évidements longitudinaux orientés dans la même direction, celle du plan médian de la cale, et destinés à recevoir les épineuses des vertèbres adjacentes entre lesquelles la cale sera implantée. La cale est ensuite maintenue en position par des sangles entourant lesdites épineuses. En bloquant une partie du rachis, la cale produit un transfert de charge au-dessus et en dessous des vertèbres concernées et soulage ainsi le disque intervertébral situé entre ces vertèbres. Or, du fait de l'anatomie de la région sacrée, et plus particulièrement de l'absence d'épineuse sur la vertèbre S1, la mise en place de ce type de cale est impossible au niveau de l'articulation L5-S1.

On connaît également un type d'implant décrit dans le document EP 1 138 268, adapté cette fois à l'anatomie de la région lombo-sacrée. Cet implant comprend une cale intervertébrale et une barre de liaison. La cale intervertébrale présente deux évidements sensiblement orthogonaux entre eux, et la barre de liaison est rendue solidaire du sacrum au moyen de deux crochets fixés à la vertèbre S1. Plus précisément, ces crochets prennent appui sur la partie supérieure de la vertèbre S1, appelée également arc postérieur, et sont fixés sur le sacrum chacun par des moyens de fixation, comme des agrafes, qui permettent de positionner et de stabiliser les crochets. Une fois les crochets installés sur le sacrum, la barre est fixée aux crochets, et la cale intervertébrale est mise en place. L'évidement supérieur de la cale est apte à recevoir l'épineuse de la vertèbre L5 et l'évidement inférieur présente une forme appropriée pour recevoir la barre, de sorte que la cale repose sur cette barre.

Cependant, ce type d'implant présente certains inconvénients. Tout d'abord, pour permettre l'engagement de l'encoche inférieure de la cale sur la barre de liaison, il est nécessaire que celle-ci soit suffisamment écartée du sacrum. Il en résulte que d'une part l'implant est relativement encombrant et que d'autre part la liaison entre l'extrémité inférieure de la cale et la barre est incertaine. En outre et surtout la mise en place de la cale entre la barre de liaison et l'apophyse épineuse de la cinquième vertèbre lombaire par le chirurgien n'est pas aisée. En effet, pour assurer un maintien dans l'apophyse et sur la barre de liaison des extrémités de la cale, il est nécessaire que les deux encoches disposées dans les extrémités de la cale soient suffisamment profondes. Il est donc nécessaire de réaliser un écartement relativement important du sacrum et de la cinquième vertèbre lombaire.

Pour remédier à ces inconvénients, un objet de la présente invention est de fournir un implant intervertébral pour l'articulation lombo-sacrée qui soit d'une tenue améliorée et qui facilite l'action des chirurgiens pour la mise en place dudit implant.

Pour atteindre ce but, selon l'invention, l'implant destiné à être placé entre le sacrum d'un patient et la cinquième vertèbre lombaire, comprend une barre de liaison, des moyens pour fixer chaque extrémité de la barre de liaison sur le sacrum, et une cale dont une première extrémité coopère avec l'apophyse épineuse de la cinquième vertèbre lombaire et une deuxième extrémité coopère avec ladite barre de liaison, l'implant se caractérisant en ce que ladite barre de liaison comprend deux extrémités présentant un même premier axe géométrique et une partie médiane raccordée auxdites extrémités, ladite partie médiane ayant un deuxième axe géométrique parallèle au premier axe géométrique mais décalé par rapport à celui-ci, ladite deuxième extrémité de la cale coopérant avec ladite partie médiane.

On comprend que, grâce à la forme particulière de la barre de liaison qui a en quelque sorte la forme d'un vilebrequin, il est possible de donner au moment de la mise en place de la cale une position convenable à la partie médiane de la barre de liaison pour en faciliter la mise en place.

Selon un mode préféré de mise en oeuvre, l'implant est caractérisé en ce que les extrémités de ladite barre de liaison ont une section droite circulaire et en ce que chaque moyen de fixation de la barre de liaison sur le sacrum comporte une tête apte à recevoir à rotation une des extrémités de ladite barre et un organe de verrouillage apte, en position active, à immobiliser en rotation ladite extrémité de la barre.

On comprend que, grâce à la combinaison des caractéristiques énoncées précédemment, il est possible de donner initialement à la barre de liaison une orientation angulaire de sa partie médiane écartée du sacrum pour faciliter la mise en place de l'extrémité inférieure de la cale, puis de provoquer la rotation de cette partie médiane autour de l'axe principal de la barre de liaison pour amener celle-ci dans la position correcte.

De préférence, l'implant est caractérisé en ce que ladite deuxième extrémité de la cale comporte un évidement pour recevoir la partie médiane de la barre, ledit évidement autorisant une libre rotation de ladite cale autour du deuxième axe géométrique de la partie médiane de la barre.

On comprend également que, grâce à la combinaison des caractéristiques énoncées précédemment, il est possible non seulement de faire pivoter la barre de liaison autour de son axe principal pour amener sa partie médiane dans une position finale convenable, mais il est également possible de provoquer le pivotement de l'ensemble de la cale autour de la partie médiane de la barre de liaison pour amener la partie supérieure de la cale en contact avec l'apophyse épineuse de la cinquième vertèbre lombaire.

De préférence encore, l'évidement prévu à l'extrémité inférieure de la cale comporte des moyens de clipsage de la partie médiane de la barre sur l'extrémité inférieure de la cale, ce qui simplifie encore l'intervention du chirurgien.

D'autres caractéristiques et avantages de l'invention apparaîtront mieux à la lecture de la description qui suit de plusieurs modes de réalisation de l'invention donnés à titre d'exemples non limitatifs. La description se réfère aux figures annexées, sur lesquelles :
- la figure 1 déjà décrite montre l'articulation lombo-sacrée ;
- la figure 2A est une vue de dessus de l'implant selon l'invention ;
- la figure 2B est une vue en élévation de l'implant ;
- la figure 2C est une vue de côté de l'implant ;
- la figure 3 est une vue en élévation de deux types de vis utilisés dans l'implant selon l'invention ;
- les figures 4A et 4B sont des vues en perspective de la cale selon deux angles différents ; et
- la figure 5 est une vue en perspective de la barre de liaison.

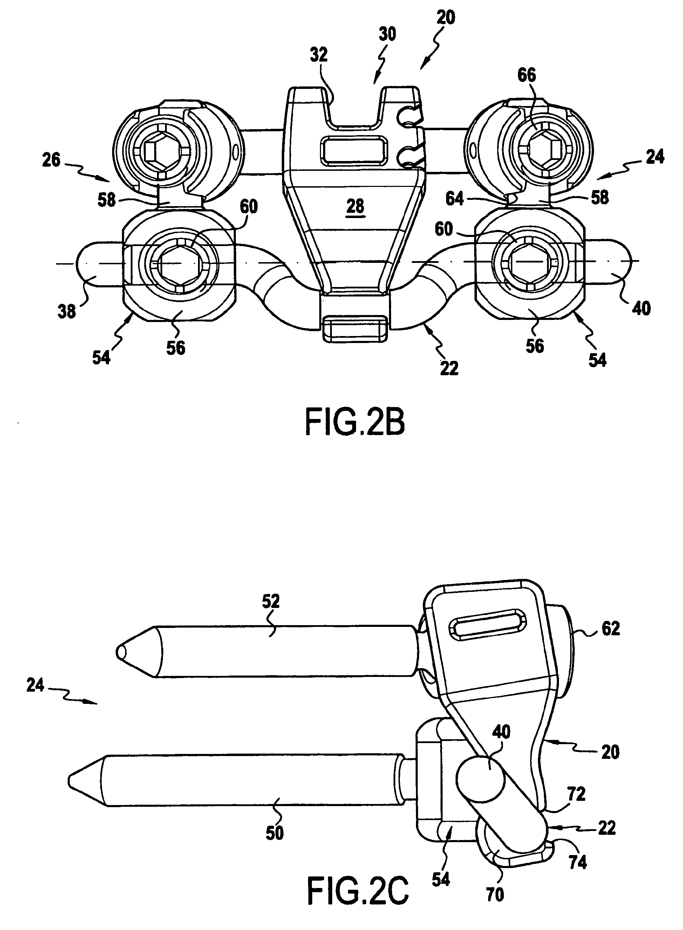

En se référant tout d'abord aux figures 2A, 2B et 2C, on va décrire les éléments constitutifs de l'implant intervertébral pour l'articulation lombo-sacrée.

L'implant est essentiellement constitué par une cale intervertébrale 20, une barre de liaison 22 et deux systèmes 24 et 26 de fixation de la barre de liaison 22 sur le sacrum.

La cale 20 comporte un corps 28, une extrémité supérieure 30 de forme standard pour une cale intervertébrale, c'est-à-dire munie d'un évidement 32 destiné à recevoir l'apophyse épineuse 10 de la cinquième vertèbre lombaire et une extrémité inférieure 34 de coopération avec la barre de liaison 22.

La barre de liaison 22 comporte une partie médiane 36 qui coopère avec la deuxième extrémité 34 de la cale intervertébrale 20 et deux extrémités de fixation 38 et 40 qui coopèrent respectivement avec les systèmes de fixation dans le sacrum 24 et 26.

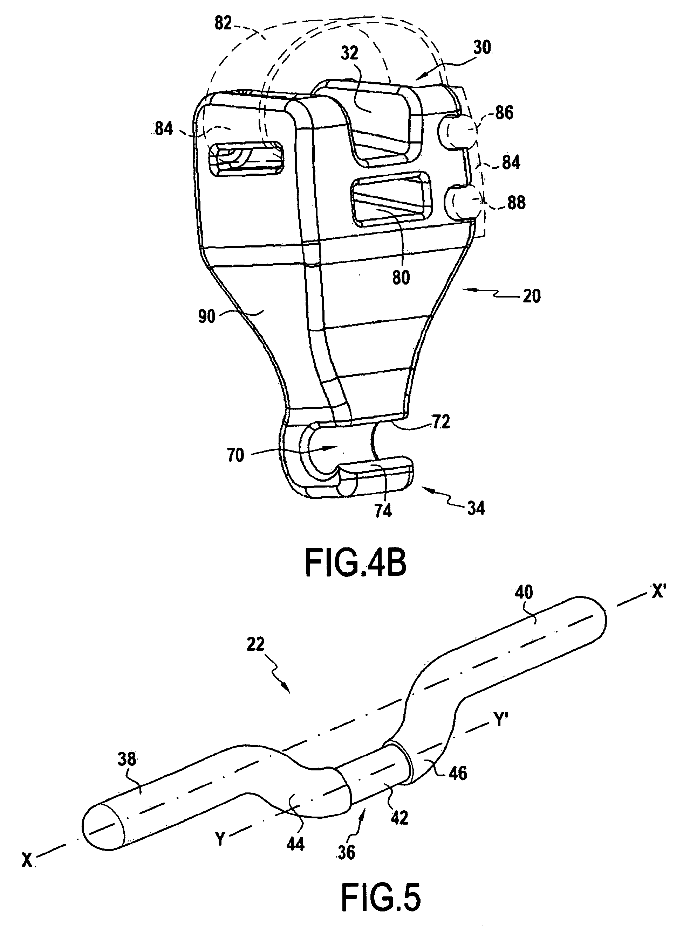

En se référant maintenant à la figure 5, on va décrire plus en détail un mode préféré de réalisation de la barre de liaison 22 qui constitue un élément essentiel de l'invention. Les extrémités de fixation 38 et 40 de la barre sont à section droite circulaire et ces extrémités sont alignées sur un même axe géométrique X, X'. En revanche, la partie médiane 36 de la barre qui coopère avec l'extrémité inférieure de la cale 20 présente un axe Y, Y' parallèle à l'axe X, X' mais décalé par rapport à celui-ci. Plus précisément, la partie médiane 36 comporte une partie cylindrique de diamètre réduit 42 et deux parties doublement coudées 44 et 46 de raccordement aux extrémités de fixation 38 et 40.

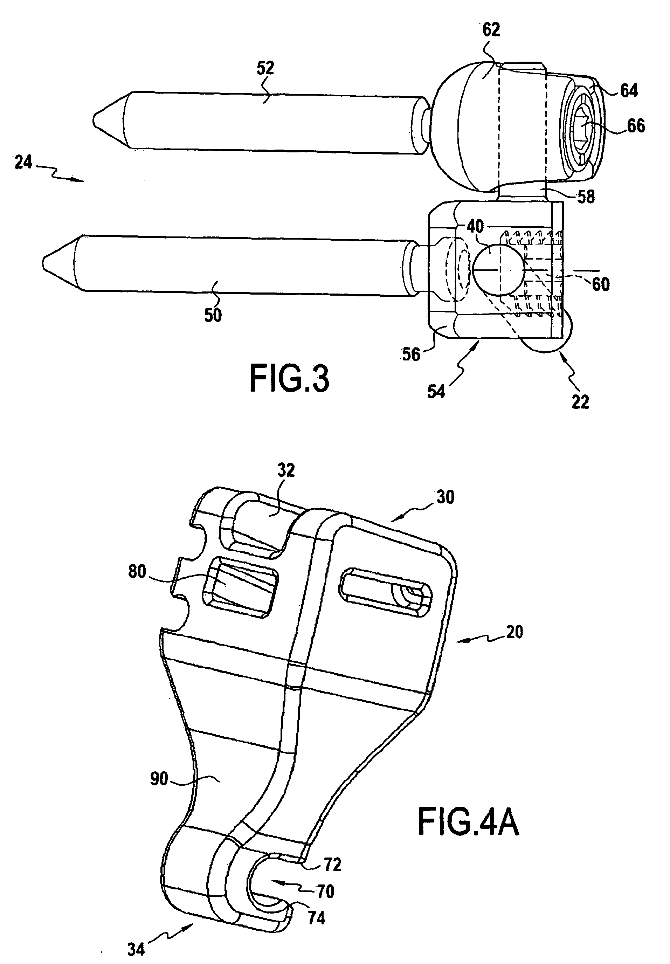

En se référant maintenant aux figures 2A, 2B, 2C et 3, on va décrire un mode de réalisation préféré des dispositifs de fixation 24 et 26 de la barre de liaison 22 sur le sacrum. Les deux dispositifs de fixation étant identiques, on décrira seulement le dispositif 24.

Celui-ci est essentiellement constitué, dans le mode de réalisation représenté sur les figures, par une vis à os inférieure 50 et une vis supérieure 52. La vis à os inférieure 50 est équipée d'un sabot 54 qui comporte une tête 56 et une portion de tige de liaison 58. La tête 56 du sabot 54 comporte un évidement semi-cylindrique apte à recevoir l'extrémité de fixation 40 de la barre de liaison 22. Un organe de verrouillage 60 lorsqu'il est serré, permet d'immobiliser l'extrémité de la barre de liaison 22 en rotation par rapport à la vis 50. La tête 62 de la vis supérieure 52 est également munie d'un évidement semi-cylindrique 64 apte à recevoir l'extrémité de la tige de liaison 58 du sabot 54. Un deuxième organe de verrouillage 66 permet d'immobiliser la tige de liaison 58 par rapport à la tête de la vis 52. Les ensembles 24 et 26 de fixation constituent des éléments d'ancrage dans le sacrum constitués essentiellement chacun par les vis 50 et 52 et par le sabot 54. On assure ainsi un ancrage très efficace des extrémités de la barre de liaison 22 dans le sacrum. Il va cependant de soi que les dispositifs de fixation 24 et 26 pourraient être simplifiés sans que l'on sorte de l'invention. Ils pourraient se réduire à une vis à os 52 pour chaque dispositif de fixation remplaçant la vis à os 50. Les extrémités de fixation 38 et 40 de la barre de liaison 22 étant alors directement engagées dans les têtes des vis à os 52.

En se référant maintenant plus particulièrement aux figures 4A et 4B, on va décrire l'extrémité inférieure 34 de la cale intervertébrale 20.

Ainsi qu'on l'a déjà indiqué, l'extrémité inférieure 34 de la cale coopère avec la partie médiane 36 de la barre de liaison 22. De préférence, l'extrémité inférieure 34 de la cale comporte un logement de section droite sensiblement circulaire 70 muni d'une fente latérale 72 pour permettre l'insertion de la partie médiane 36 de la barre de liaison et, plus précisément, de sa portion de diamètre réduit 42. Un des bords de la fente d'insertion 72 constitue un bec de clipsage 74 de telle manière que la partie de diamètre réduit 42 puisse être engagée dans le logement 70 par déformation élastique du bec 74. En outre, le diamètre de la section droite du logement 70 est très légèrement supérieur au diamètre de la portion de diamètre réduit 42 de la barre de liaison. Ainsi, après l'engagement dans le logement 70 de la portion 42 de la barre de liaison, la cale 20 peut pivoter librement en rotation autour du deuxième axe Y, Y' de la barre. Cependant, grâce à la présence du bec 74, la cale reste clipsée sur la barre par son extrémité inférieure.

L'extrémité supérieure 30 de la cale 20 comporte essentiellement un évidement 32, ainsi qu'on l'a déjà indiqué, destiné à recevoir l'apophyse épineuse 10 de la vertèbre lombaire L5. Comme le montrent les figures 4A et 4B, de préférence le corps de la cale comporte un évidement traversant celui-ci de part en part et référencé 80 dont la fonction est de conférer une certaine élasticité au corps de la cale intervertébrale. Comme cela est en soi bien connu, il est nécessaire de solidariser l'extrémité supérieure 30 de la cale et l'apophyse épineuse 10. Pour cela de préférence, on prévoit un lien souple 82 dont une première extrémité 84 est solidaire d'un des bords de l'évidement 32. Sur la face opposée du corps de la cale, est prévu un dispositif autobloquant 84 fixé sur la cale de préférence par un système de clipsage 86, 88. L'extrémité libre du lien 82 est engagée dans le dispositif autobloquant 84 et par une traction sur cette extrémité libre, le chirurgien assure la fixation de l'apophyse épineuse dans le logement supérieur 32.

On va maintenant décrire les étapes de mise en place de l'implant par le chirurgien.

Dans un premier temps, le chirurgien visse dans le sacrum les vis à os inférieures 50, les vis à os supérieures 52 et il monte sur les têtes des vis inférieures 50 les sabots 54. Les verrous 66 des vis supérieures sont serrés de telle manière qu'on obtienne effectivement la solidarisation entre les vis 50 et les vis 52 via les sabots 54. Dans l'étape suivante, les extrémités de fixation 38 et 40 de la barre de liaison 22 sont engagées dans les évidements semi-cylindriques des sabots 54. Les extrémités 38 et 40 de la barre étant à section droite circulaire, la partie médiane 36 de la barre peut librement pivoter autour de l'axe X, X' de cette même barre par rapport aux dispositifs de fixation 24 et 26. Grâce à cette possibilité de rotation, la partie médiane 36 de la barre peut être écartée du sacrum pour faciliter l'engagement de la partie de diamètre réduit 42 de la barre dans l'évidement de clipsage 70. Après cet engagement de l'extrémité inférieure de la cale sur la barre avec clipsage, il est possible de faire pivoter la partie médiane de la barre de telle manière que la face antérieure 90 du corps de la cale soit effectivement en appui sur le sacrum. Comme le montrent les figures 4A et 4B, la face antérieure 90 du corps de la cale n'est pas plane. Elle est conformée pour s'adapter au mieux à la forme du sacrum.

En outre, du fait que la cale 20 peut librement pivoter autour de l'axe Y, Y' de la partie médiane de la barre 22, l'engagement de l'apophyse épineuse de la cinquième vertèbre lombaire dans le logement supérieur 32 de la cale est grandement facilité. Cette double possibilité de pivotement de la cale par rapport à la barre et de la barre par rapport aux dispositifs de fixation dans le sacrum 24 et 26 permet d'assurer un positionnement optimum de la cale 20. En particulier, l'extrémité inférieure de la cale peut être "coincée" entre la partie médiane 36 de la barre engagée dans le logement 70 et la face postérieure du sacrum. Lorsque la position convenable de la cale 20 est obtenue et par voie de conséquence, la position convenable de la barre de liaison 22, les organes de verrouillage 60 des sabots 54 sont serrés de telle manière que la barre 22 soit immobilisée par rapport au dispositif de fixation 24 et 26 et donc par rapport au sacrum.

Il ne reste plus alors au chirurgien qu'à mettre en place le lien 82 au-dessus de l'apophyse épineuse et d'exercer une traction sur l'extrémité libre de ce lien pour obtenir le serrage du lien autour de cette apophyse et donc la solidarisation de l'extrémité supérieure de la cale 20 avec cette apophyse.

## Revendications

1. Implant destiné à être placé entre le sacrum d'un patient et la cinquième vertèbre lombaire, comprenant une barre de liaison (22), des moyens pour fixer chaque extrémité de la barre de liaison sur le sacrum (24, 26), et une cale (20) dont une première extrémité coopère avec l'apophyse épineuse de la cinquième vertèbre lombaire et une deuxième extrémité (34) coopère avec ladite barre de liaison, **caractérisé en ce que**
ladite barre de liaison comprend deux extrémités (38, 40) présentant un même premier axe géométrique (X-X') et une partie médiane (36) raccordée auxdites extrémités, ladite partie médiane ayant un deuxième axe géométrique (Y-Y') parallèle au premier axe géométrique mais décalé par rapport à celui-ci, ladite deuxième extrémité (34) de la cale coopérant avec ladite partie médiane.

2. Implant selon la revendication 1, **caractérisé en ce que** les extrémités de ladite barre de liaison ont une section droite circulaire et **en ce que** chaque moyen de fixation de la barre de liaison sur le sacrum comporte une tête (56) apte à recevoir à rotation une des extrémités de ladite barre (38, 40) et un organe de verrouillage (60) apte, en position active, à immobiliser en rotation ladite extrémité de la barre.

3. Implant selon l'une quelconque des revendications 1 et 2, **caractérisé en ce que** ladite deuxième extrémité de la cale comporte un évidement (70) pour recevoir la partie médiane de la barre, ledit évidement autorisant une libre rotation de ladite cale autour du deuxième axe géométrique de la partie médiane de la barre.

4. Implant selon la revendication 3, **caractérisé en ce que** ladite deuxième extrémité (34) de la cale comprend des moyens de clipsage de la cale sur la partie médiane (36) de la barre.

5. Implant selon l'une quelconque des revendications 1 à 4, **caractérisé en ce que** ladite cale comprend une première face tournée vers la colonne vertébrale et une deuxième face opposée à la première, ladite première face étant non plane pour se conformer à la forme du sacrum sur lequel elle s'appuie.

6. Implant selon la revendication 2, **caractérisé en ce que** chaque moyen de fixation comprend en outre une partie filetée (50, 52) de vissage dans le sacrum et **en ce que** la tête comporte un évidement ouvert semi-cylindrique pour recevoir une extrémité cylindrique de ladite barre.

7. Implant selon la revendication 3, **caractérisé en ce que** l'évidement (70) prévu à la deuxième extrémité de ladite cale a une section suffisante pour recevoir librement à rotation la partie médiane de la barre et **en ce que** la cale comporte en outre une fente d'insertion de la barre dans l'évidement, ménagée dans la deuxième face de la cale, la dimension de ladite fente par rapport à la section droite de la partie médiane étant telle qu'elle produit un effet de clipsage.

## Claims

1. An implant intended to be placed between the sacrum of a patient and the fifth lumbar vertebra, the implant comprising a connection rod (22), means for fastening each end of the connection rod to the sacrum (24, 26), and a spacer (20) having a first end for co-operating with the spinous process of the fifth lumbar vertebra, and a second end (34) for co-operating with said connection rod, the implant being **characterized in that**
said connection rod has two ends (38, 40) lying on a common first geometrical axis (X-X') and a middle portion (36) connected to said ends, said middle portion having a second geometrical axis (Y-Y') parallel to the first geometrical axis but offset relative thereto, the second end (34) of the spacer co-operating with said middle portion.

2. An implant according to claim 1, **characterized in that** the ends of said connection rod are of circular right section and **in that** each fastener means for fastening the connection rod to the sacrum includes a head (56) suitable for pivotally receiving one of the ends of said rod (38, 40) and a locking member (60) suitable, in its active position, for preventing said end of the rod from pivoting.

3. An implant according to claim 1 or claim 2, **characterized in that** said second end of the spacer includes a recess (70) for receiving the middle portion of the rod, said recess allowing said spacer to pivot freely about the second geometrical axis of the middle portion of the rod.

4. An implant according to claim 3, **characterized in that** said second end (34) of the spacer includes snap-fastener means for fastening the spacer on the middle portion (36) of the rod.

5. An implant according to any one of claims 1 to 4, **characterized in that** said spacer has a first face facing towards the vertebral column and a second face, opposite from the first, said first face being non-plane so as to match the shape of the sacrum against which it bears.

6. An implant according to claim 2, **characterized in that** each fastener means further comprises a threaded portion (50, 52) for screwing into the sacrum, and **in that** the head includes an open semicylindrical recess for receiving a cylindrical end of said rod.

7. An implant according to claim 3, **characterized in that** the recess (70) provided at the second end of said spacer is of section that is sufficient to receive the middle portion of the rod to pivot freely, and **in that** the spacer further includes a slot for inserting the rod into the recess, the slot being formed in the second face of the spacer and being of a size relative to the right section of the middle portion that is such as to produce a snap-fastening effect.

## Patentansprüche

1. Implantat, das dazu bestimmt ist, zwischen dem Kreuzbein eines Patienten und dem fünften Lendenwirbel plaziert zu werden, umfassend einen Verbindungsstab (22), Mittel zum Befestigen eines jeden Endes des Verbindungsstabes an dem Kreuzbein (24, 26) sowie einen Keil (20), von dem ein erstes Ende mit dem Dornfortsatz des fünften Lendenwirbels zusammenwirkt und ein zweites Ende (34) mit dem Verbindungsstab zusammenwirkt, **dadurch gekennzeichnet, daß**
der Verbindungsstab zwei Enden (38, 40) mit einer gleichen ersten geometrischen Achse (X-X') und einen mit den Enden verbundenen Mittelteil (36) umfaßt, wobei der Mittelteil eine zweite geometrische Achse (Y-Y') aufweist, die zu der ersten geometrischen Achse parallel verläuft, aber zu dieser versetzt ist, wobei das zweite Ende (34) des Keils mit dem Mittelteil zusammenwirkt.

2. Implantat nach Anspruch 1, **dadurch gekennzeichnet, daß** die Enden des Verbindungsstabes einen kreisförmigen Querschnitt aufweisen und daß jedes Mittel zum Befestigen des Verbindungsstabes an dem Kreuzbein einen Kopf (56), der geeignet ist, eines der Enden des Stabes (38, 40) drehbar aufzunehmen, sowie ein Verriegelungsorgan (60) umfaßt, das geeignet ist, in der aktiven Position das Ende des Stabes gegen ein Drehen festzulegen.

3. Implantat nach einem der Ansprüche 1 und 2, **dadurch gekennzeichnet, daß** das zweite Ende des Keils eine Ausnehmung (70) zur Aufnahme des Mittelteils des Stabes aufweist, wobei die Ausnehmung ein freies Drehen des Keils um die zweite geometrische Achse des Mittelteils des Stabes zuläßt.

4. Implantat nach Anspruch 3, **dadurch gekennzeichnet, daß** das zweite Ende (34) des Keils Mittel zum Festclipsen des Keils an dem Mittelteil (36) des Stabes umfaßt.

5. Implantat nach einem der Ansprüche 1 bis 4, **dadurch gekennzeichnet, daß** der Keil eine der Wirbelsäule zugewandte erste Seite und eine der ersten gegenüberliegende zweite Seite umfaßt, wobei die erste Seite nicht eben ist, um sich der Form des Kreuzbeins, an dem sie anliegt, anzupassen.

6. Implantat nach Anspruch 2, **dadurch gekennzeichnet, daß** jedes Befestigungsmittel ferner einen Gewindeteil (50, 52) zum Einschrauben in das Kreuzbein umfaßt und daß der Kopf eine halbzylindrische, offene Ausnehmung zur Aufnahme eines zylindrischen Endes des Stabes umfaßt.

7. Implantat nach Anspruch 3, **dadurch gekennzeichnet, daß** die an dem zweiten Ende des Keils vorgesehene Ausnehmung (70) einen Querschnitt aufweist, der ausreichend ist, um den Mittelteil des Stabes drehfrei aufzunehmen, und daß der Keil ferner einen Schlitz zum Einführen des Stabes in die Ausnehmung aufweist, der in der zweiten Seite des Keils ausgebildet ist, wobei die Abmessung des Schlitzes gegenüber dem Querschnitt des Mittelteils derart ist, daß sie eine Festclipswirkung erzeugt.
